# EUROPÄISCHE PATENTSCHRIFT

(11) **EP 1 496 902 B1**
(45) Veröffentlichungstag und Bekanntmachung des Hinweises auf die Patenterteilung: **14.12.2005**
(21) Anmeldenummer: 03712137.3
(22) Anmeldetag: 09.04.2003
(51) Int. Cl.: A61K 31/46, A61K 31/56, A61P 11/06, A61K 9/12

(54) **ARZNEIMITTEL ENTHALTEND STEROIDE UND EIN NEUES ANTICHOLINERGIKUM**
MEDICAMENT CONTAINING STEROIDS AND A NOVEL ANTICHOLINERGIC DRUG
MEDICAMENT CONTENANT DES STEROIDES ET UN NOUVEL ANTICHOLINERGIQUE

(30) Priorität: 12.04.2002 DE 10216429
(43) Veröffentlichungstag der Anmeldung: 19.01.2005
(60) Teilanmeldung: 05109887.9
(73) Patentinhaber: Boehringer Ingelheim Pharma GmbH & Co.KG, 55216 Ingelheim am Rhein (DE)
(72) Erfinder: BANHOLZER, Rolf, 70597 STUTTGART (DE); MEADE, Christopher, John, Montague, 88437 Maselheim (DE); MEISSNER, Helmut, 55218 INGELHEIM (DE); MORSCHHÄUSER, Gerd, 88400 BIBERACH (DE); PAIRET, Michel, 88400 BIBERACH (DE); PIEPER, Michael, P., 88400 BIBERACH (DE); POHL, Gerald, 88400 BIBERACH (DE); REICHL, Richard, 55435 GAU-ALGESHEIM (DE); SPECK, Georg, 55218 INGELHEIM AM RHEIN (DE)
(86) Internationale Anmeldenummer: PCT/EP2003/003668
(87) Internationale Veröffentlichungsnummer: WO 2003/086399

(56) Entgegenhaltungen:
- EP-A- 0 823 423
- WO-A-01/78745
- WO-A-02/32899
- WO-A-02/36106

## Beschreibung

Die vorliegende Erfindung betrifft neuartige Arzneimittelkompositionen auf der Basis von langwirksamen Steroiden und Salzen eines neuen Anticholinergikums, Verfahren zu deren Herstellung sowie deren Verwendung bei der Therapie von Atemwegserkrankungen.

### Beschreibung der Erfindung

Die vorliegende Erfindung betrifft neuartige Arzneimittelkompositionen auf der Basis von Steroiden und Salzen eines neuen Anticholinergikums **1****,** Verfahren zu deren Herstellung sowie deren Verwendung bei der Therapie von Atemwegserkrankungen.

Im Rahmen der vorliegenden Erfindung gelangen als Anticholinergikum die Salze der Formel **1** 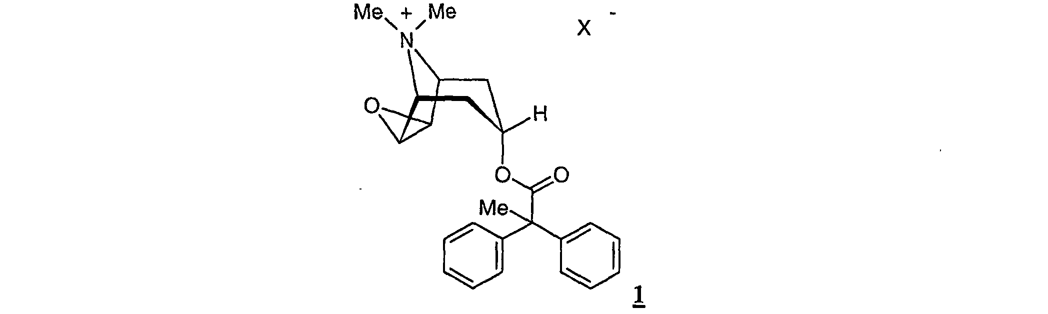 worin
- X⁻: ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet zur Anwendung.

Bevorzugt gelangen die Salze der Formel **1** zur Anwendung, worin
- X⁻: ein einfach negativ geladenes Anion ausgewählt aus der Gruppe Chlorid, Bromid, 4-Toluolsulfonat und Methansulfonat, bevorzugt Bromid, bedeutet.

Besonders bevorzugt gelangen die Salze der Formel **1** zur Anwendung, worin
- X⁻: ein einfach negativ geladenes Anion ausgewählt aus der Gruppe Chlorid, Bromid und Methansulfonat, bevorzugt Bromid, bedeutet.

Erfindungsgemäß besonders bevorzugt ist dasjenige Salz der Formel **1**, in dem X - für Bromid steht.

Anticholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden durch die WO 92/16528 Anticholinergika vorgeschlagen, die ein Scopin-, Tropenol- oder auch Tropin-Grundgerüst aufweisen.

Die der WO 92/16528 zugrunde liegende Aufgabe zielt auf die Bereitstellung von anticholinerg wirksamen Verbindungen, die durch eine lang andauernde Wirksamkeit gekennzeichnet sind. Zur Lösung dieser Aufgabe werden durch die WO 92/16528 unter anderem Benzilsäureester des Scopins, Tropenols oder auch Tropins offenbart.

Zur Therapie chronischer Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Um als einmal täglich anwendbares Medikament zum Einsatz kommen zu können, sind an den zu applizierenden Wirkstoff besondere Anforderungen zu stellen. Zunächst sollte der nach Gabe des Arzneimittels erwünschte Wirkungseintritt relativ schnell erfolgen und im Idealfall über einen sich daran anschließenden längeren Zeitraum eine möglichst konstante Wirksamkeit aufweisen. Andererseits sollte die Wirkdauer des Arzneimittels einen Zeitraum von etwa einem Tag nicht wesentlich überschreiten. Im Idealfall zeigt ein Wirkstoff ein derart geartetes Wirkungsprofil, daß sich die Herstellung eines einmal täglich applizierbaren Arzneimittels, welches den Wirkstoff in therapeutisch sinnvollen Dosen enthält, gezielt steuern läßt.

Es wurde gefunden, daß die in der WO 92/16528 offenbarten Benzilsäureester des Scopins, Tropenols oder auch Tropins diesen erhöhten Anforderungen nicht genügen. Sie sind aufgrund ihrer extrem langen Wirkungsdauer, die den vorstehend genannten Zeitraum von etwa einem Tag deutlich überschreiten, nicht als Einmalgabe pro Tag therapeutisch nutzbar.

Überraschenderweise kann ferner ein unerwartet vorteilhafter therapeutischer Effekt, insbesondere ein synergistischer Effekt bei der Behandlung von entzündlichen oder obstruktiven Atemwegserkrankungen beobachtet werden, wenn das Anticholinergikum der Formel **1** gemeinsam mit einem oder mehreren, bevorzugt einem Steroid **2** zur Anwendung gelangen. Aufgrund dieses synergistischen Effekts sind die erfindungsgemäßen Arzneimittelkombinationen in geringerer Dosierung einsetzbar, als dies bei der sonst üblichen Monotherapie der Einzelverbindungen der Fall ist. Dadurch lassen sich gegebenenfalls unerwünschte Nebenwirkungen, wie sie beispielsweise bei der Applikation von Steroiden auftreten können, vermindern.

Die vorstehend genannten Effekte können sowohl bei gleichzeitiger Applikation innerhalb einer einzigen Wirkstoffformulierung als auch bei sukzessiver Applikation der beiden Wirkstoffe in getrennten Formulierungen beobachtet werden. Erfindungsgemäß bevorzugt ist die gleichzeitige Applikation der beiden Wirkstoffbestandteile in einer einzigen Formulierung. Die erfindungsgemäßen Arzneimittel werden erfindungsgemäß bevorzugt inhalativ appliziert.

Eine gegebenenfalls im Rahmen der vorliegenden Erfindung erfolgende Bezugnahme auf die Verbindung **1'** ist als Bezugnahme auf das in den Salzen **1** enthaltene pharmakologisch aktive Kation der nachstehenden Formel anzusehen

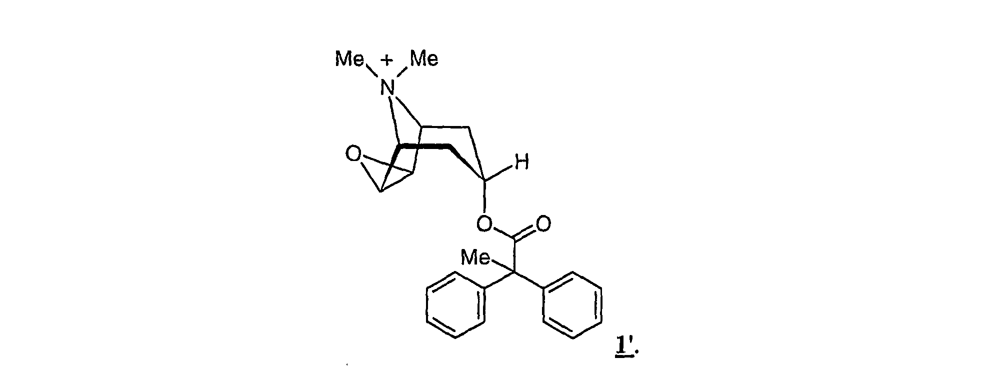

Eine Bezugnahme auf Verbindungen **1** schließt naturgemäß eine Bezugnahme auf das Kation **1'** mit ein.

Bei den vorstehend genannten Arzneimittelkombinationen können die Wirkstoffe **1** und **2** entweder gemeinsam in einer einzigen Darreichungsform oder in zwei getrennten Darreichungsformen enthalten sein. Erfindungsgemäß bevorzugt sind Arzneimittel, die die Wirkstoffe **1** und **2** in einer einzigen Darreichungsform enthalten.

Im Rahmen der vorliegenden Erfindung werden unter Steroiden (im Folgenden **2**), die nachfolgend gegebenenfalls auch als Corticosteroide bezeichnet werden, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, ST-126 und Dexametasone. Bevorzugt ist die Verbindung **2** ausgewählt aus der Gruppe bestehend aus Budesonid, Fluticasone, Mometasone, Ciclesonide und ST-126.

Eine Bezugnahme auf Steroide **2** schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Verbindungen der Formel **2** auch in Form ihrer Hydrate vorliegen. Eine Bezugnahme auf Steroide **2** schließt im Rahmen der vorliegenden Erfindung ferner eine Bezugnahme auf die Verbindungen **2** in Form ihrer Diastereomere, Gemische der Diastereomeren oder in Form der Racemate ein.

Ein Aspekt der vorliegenden Erfindung betrifft vorstehend genannte Arzneimittel, welche neben therapeutisch wirksamen Mengen von **1** und **2** einen pharmazeutisch verträglichen Trägerstoff enthalten. Ein weiterer Aspekt der vorliegenden Erfindung betrifft vorstehend genannte Arzneimittel, welche neben therapeutisch wirksamen Mengen von **1** und **2** keinen pharmazeutisch verträglichen Trägerstoff enthalten.

Die vorliegende Erfindung betrifft ferner die Verwendung therapeutisch wirksamer Mengen der Salze **1** zur Herstellung eines ferner Steroide **2** enthaltenden Arzneimittels zur Behandlung von entzündlichen oder obstruktiven Atemwegserkrankungen. Bevorzugt betrifft die vorliegende Erfindung die vorstehend genannte Verwendung zur Herstellung eines Arzneimittels zur Behandlung von Asthma oder COPD.

Im Rahmen der vorliegenden Erfindung kann die Applikation der Verbindungen **1** und **2** gleichzeitig oder nacheinander erfolgen, wobei die gleichzeitige Gabe der Verbindungen **1** und **2** erfindungsgemäß bevorzugt ist.

Die vorliegende Erfindung betrifft ferner die Verwendung therapeutisch wirksamer Mengen an Salzen **1** und Steroiden **2** zur Behandlung von entzündlichen oder obstruktiven Atemwegserkrankungen, insbesondere von Asthma oder COPD.

Die Verhältnisse, in denen die Wirkstoffe **1** und **2** in die erfindungsgemäßen Wirkstoffkombinationen eingesetzt werden können, sind variabel. Die Wirkstoffe **1** und **2** können gegebenfalls in Form ihrer Solvate oder Hydrate vorliegen. Je nach Wahl der Verbindungen **1** und **2** variieren die im Rahmen der vorliegenden Erfindung einsetzbaren Gewichtsverhältnisse aufgrund des unterschiedlichen Molekulargewichts der verschiedenen Verbindungen sowie aufgrund ihrer unterschiedlichen Wirkstärke. In der Regel können die erfindungsgemäßen Arzneimittelkombinationen das Kation **1**' und ein Steroid **2** in Gewichtsverhältnissen enthalten, die in einem Bereich von 1:250 bis 250:1, bevorzugt von 1:150 bis 150:1, liegen. Bei den besonders bevorzugten Arzneimittelkompositionen, die neben **1**' eine Verbindung ausgewählt aus der Gruppe bestehend aus Budesonid, Fluticasone, Mometasone, ST-126 und Ciclesonide als Steroid **2** enthalten, liegen die Gewichtsverhältnisse von **1****'** zu **2** besonders bevorzugt in einem Bereich von 1:40 bis 40:1, ferner bevorzugt von 1:30 bis 30:1.

Beispielsweise, können bevorzugte erfindungsgemäße Kombinationen aus **1** und **2** das Kation **1**' und eines der vorstehend genannten bevorzugt zur Anwendung gelangenden Steroide **2** in den folgenden Gewichtsverhältnissen enthalten: 1:20; 1:19; 1:18; 1:17; 1:16; 1:15; 1:14; 1:13; 1:12; 1:11; 1:10; 1:9; 1:8; 1:7; 1:6; 1:5; 1:4; 1:3; 1:2; 1:1; 2:1; 3:1; 4:1; 5:1; 6:1; 7:1; 8:1; 9:1; 10:1; 11:1; 12:1; 13:1; 14:1; 15:1; 16:1; 17:1; 18:1; 19:1; 20:1.

Die Anwendung der erfindungsgemäßen Arzneimittel enthaltend die Kombinationen aus **1** und **2** erfolgt üblicherweise so, dass **1'** und **2** gemeinsam in Dosierungen von 5 bis 5000µg, bevorzugt von 10 bis 2000µg, besonders bevorzugt von 15 bis 1000µg, ferner bevorzugt von 20 bis 800µg, erfindungsgemäß bevorzugt von 30 bis 700µg, bevorzugt von 40 bis 600µg, bevorzugt von 50 bis 550µg, bevorzugt von 40 bis 500µg, besonders bevorzugt von 50 bis 400µg pro Einmalgabe enthalten sind. Beispielsweise enthalten erfindungsgemäße Kombinationen aus **1** und **2** eine solche Menge an **1****'** und Steroid **2**, dass die Gesamtdosierung pro Einmalgabe etwa 35µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140 µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg beträgt. Für den Fachmann ist ersichtlich, dass vorstehend genannte Dosierungsvorschläge pro Einmalgabe nicht als auf die explizit angegebenen Zahlenwerte beschränkt anzusehen ist. Schwankungen um etwa ± 2,5µg, insbesondere Schwankungen im Dezimalbereich sind, wie für den Fachmann ersichtlich, ebenfalls umfasst. Bei diesen Dosierungsbereichen können die Wirkstoffe **1****'** und **2** in den vorhergehend beschriebenen Gewichtsverhältnissen enthalten sein.

Beispielsweise, können die erfindungsgemäßen Kombinationen aus **1** und **2** eine solche Menge an Kation **1**' und Steroid **2** enthalten, dass pro Einmalgabe 16,5µg **1**' und 25µg **2**, 16,5µg **1**' und 50µg **2**, 16,5µg **1**' und 100µg **2**, 16,5µg **1**' und 150µg **2**, 16,5µg **1**' und 200µg **2**, 16,5µg **1**' und 250µg **2**, 33,0µg **1**' und 25µg **2**, 33,0µg **1**' und 50µg **2**, 33,0µg **1**' und 100µg **2**, 33,0µg **1**' und 150µg **2**, 33,0µg **1**' und 200µg **2**, 33,0µg **1**' und 250µg **2**, 49,5µg **1**' und 25µg **2**, 49,5µg **1**' und 50µg **2**, 49,5µg **1**' und 100µg 2, 49,5µg **1**' und 150µg **2**, 49,5µg **1**' und 200µg **2**, 49,5µg **1'** und 250µg **2**, 82,6µg **1'** und 25µg **2**, 82,6µg **1'** und 50µg **2**, 82,6µg **1'** und 100µg **2**, 82,6µg **1'** und 150µg **2,** 82,6µg **1****'** und 200µg **2**, 82,6µg **1****'** und 250µg **2**, 165,1µg **1****'** und 25µg **2**, 165,1µg **1**' und 50µg **2** 165,1µg **1****'** und 50µg **2**, 165,1 µg **1'** und 100µg **2**, 165,1µg **1****'** und 150µg **2**, 165,1 µg **1****'** und 200µg **2**, 165,1µg **1****'** und 250µg **2**, 20.6,4µg **1****'** und 25µg **2**, 206,4µg **1****'** und 50µg **2**, 206,4µg **1****'** und 100µg **2**, 206,4µg **1****'** und 150µg **2**, 206,4µg **1****'** und 200µg **2**, 206,4µg **1****'** und 250µg **2**, 412,8µg **1****'** und 25µg **2**, 412,8µg **1'** und 50µg **2**, 412,8µg **1'** und 100µg **2**, 412,8µg **1****'** und 150µg **2**, 412,8µg **1****'** und 200µg **2**, 412,8µg **1****'** und 250µg **2** enthalten sind.

Wird als eine erfindungsgemäß bevorzugte Kombination aus **1** und **2** die Wirkstoffkombination verwendet, in der als Salz **1** das Bromid verwendet wird und in der **2** für eines der vorstehend als bevorzugt offenbarten Steroide steht, entsprechen die vorstehen beispielhaft genannten pro Einmalgabe applizierten Wirkstoffmengen von **1'** und **2** den nachfolgenden pro Einmalgabe applizierten Mengen an **1** und **2**: 20µg **1** und 25µg **2**, 20µg **1** und 50µg **2**, 20µg **1** und 100µg **2**, 20µg **1** und 150µg **2**, 20µg **1** und 200µg **2**, 20µg **1** und 250µg **2***,* 40µg **1** und 25µg **2**, 40µg **1** und 25µg **2**, 40µg **1** und 50µg **2**, 40µg **1** und 100µg **2**, 40µg **1** und 150µg **2**, 40µg **1** und 200µg **2**, 40µg **1** und 250µg **2**, 60µg **1** und 25µg **2**, 60µg **1** und 50µg **2**, 60µg **1** und 100µg **2**, 60µg **1** und 150µg **2**, 60µg **1** und 200µg **2**, 60µg **1** und 250µg **2**, 100µg **1** und 25µg **2**, 100µg **1** und 50µg **2**, 100µg **1** und 100µg **2*****,*** 100µg **1** und 150µg **2**, 100µg **1** und 200µg **2**, 100µg **1** und 250µg **2**, 200µg **1** und 25µg **2**, 200µg **1** und 50µg **2**, 200µg **1** und 100µg **2**, 200µg **1** und 150µg **2**, 200µg **1** und 200µg **2**, 200µg **1** und 250µg **2**, 250µg **1** und 25µg **2**, 250µg **1** und 50µg **2**, 250µg **1** und 100µg **2**, 250µg **1** und 150µg **2**, 250µg **1** und 200µg **2**, 250µg **1** und 250µg **2**, 500µg **1** und 25µg **2**, 500µg **1** und 50µg **2**, 500µg **1** und 100µg **2**, 500µg **1** und 150µg **2**, 500µg **1** und 200µg **2**, 500µg **1** und 250µg **2**.

Die Applikation der erfindungsgemäßen Wirkstoffkombinationen aus **1** und **2** erfolgt bevorzugt auf inhalativem Wege. Hierzu müssen die Bestandteile **1** und **2** in inhalierbaren Darreichungsformen bereitgestellt werden.
Als inhalierbare Darreichungsformen kommen erfindungsgemäß Inhalationspulver, treibgashaltige Dosieraerosole, treibgasfreie Inhalationslösungen oder -suspensionen in Betracht. Erfindungsgemäße Inhalationspulver enthaltend die Wirkstoffkombination aus **1**, und **2** können allein aus den genannten Wirkstoffen oder aus einem Gemisch der genannten Wirkstoffe mit physiologisch verträglichen Hilfsstoffen bestehen. Gegebenenfalls wird im Rahmen der vorliegenden Erfindung statt des Begriffs Hilfsstoffs auch der Begriff Trägerstoff verwendet. Im Rahmen der vorliegenden Erfindung sind von dem Begriff treibgasfreie Inhalationslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthaltend die erfindungsgemäßen Wirkstoffkombinationen aus 1 und 2:

Die erfindungsgemäßen Inhaltionspulver können **1** und **2** entweder allein oder im Gemisch mit geeigneten physiologisch unbedenklichen Hilfsstoffen enthalten.

Sind die Wirkstoffe **1** und **2** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhaltionspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff **1** und **2**, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhaltionspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden.
Erfindungsgemäße Inhalationspulver, die neben **1** und **2** ferner einen oder mehrere physiologisch unbedenkliche Hilfsstoffe enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Vorzugsweise werden die erfindungsgemäßen Inhalationspulver, die neben **1** und **2** physiologisch unbedenkliche Hilfsstoffe enthalten, allerdings in Kapseln abgefüllt (zu sogenannten Inhaletten), die in Inhalatoren wie beispielsweise in der WO 94/28958 beschrieben, zur Anwendung gelangen.

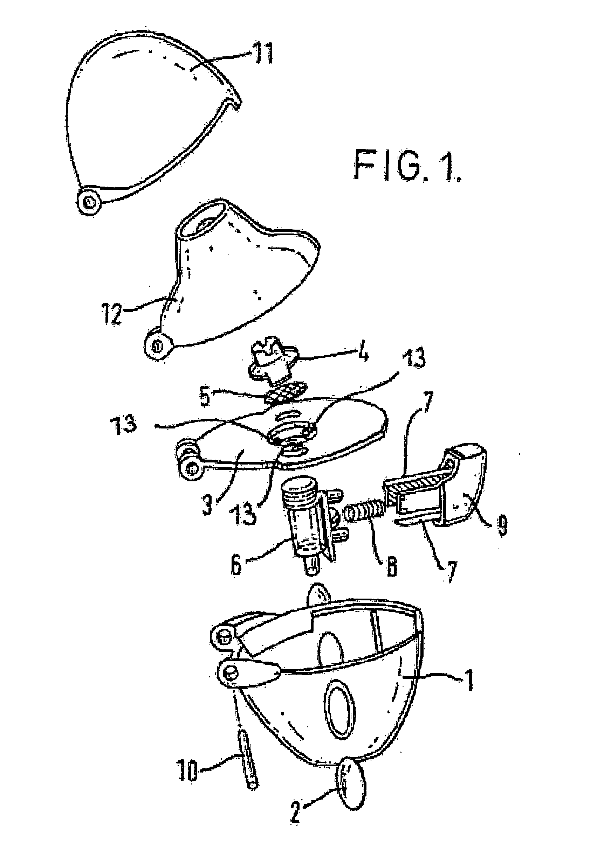

Ein zur Anwendung der erfindungsgemäßen Arzneimittelkombination in Inhaletten besonders bevorzugter Inhalator ist Figur 1 zu entnehmen.
Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlaßöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12 sowie Luftdurchlaßlöcher 13 zur Einstellung des Strömungswiderstands.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg, bevorzugt von 3 bis 20mg, bevorzugt 5 bis 10 mg Inhalationspulver pro Kapsel an. Diese enthalten erfindungsgemäß entweder gemeinsam oder jeweils die bereits vorstehend für **1** bzw. **1****'** und **2** genannten Dosierungen pro Einmalgabe.

### B) Treibgashaltige Inhalationsaerosole enthaltend die erfindungsgemäßen Wirkstoffkombinationen aus 1 und 2:

Erfindungsgemäße treibgashaltige Inhaltionsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder gegebenenfalls nur eine Komponente gelöst und die andere Komponente dispergiert enthalten sein können.
Die zur Herstellung der erfindungsgemäßen Inhaltionsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie chlorierten und/oder fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt ausTG11, TG12, TG134a (1,1,1,2-Tetrafluorethan) und TG227 (1,1,1,2,3,3,3-Heptafluorpropan) und Mischungen derselben, wobei die Treibgase TG134a, TG227 und Gemische derselben bevorzugt sind.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel, Konservierungsmittel sowie Mittel zur Einstellung des pH-Werts enthalten. All diese Bestandteile sind im Stand der Technik bekannt.

Die erfindungsgemäßen treibgashaltigen Inhaltionsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2**.

Liegen die Wirkstoffe **1** und/oder **2** in dispergierter Form vor, weisen die Wirkstoffteilchen bevorzugt eine mittlere Teilchengröße von bis zu 10 µm, bevorzugt von 0,1 bis 6 µm, besonders bevorzugt von 1 bis 5 µm auf.

Die vorstehend genannten erfindungsgemäßen treibgashaltigen Inhaltionaerosole können mittels im Stand der Technik bekannten Inhalatoren (MDIs = metered dose inhalers) appliziert werden.

Eine weitere Anwendungs möglichkeist ist die Verwendung in Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

### C) Treibgasfreie Inhaltionslösungen oder Suspensionen enthaltend die erfindungsgemäßen Wirkstoffkombinationen aus 1 und 2:

Erfindungsgemäße treibgasfreie Inhalationslösungen und Inhaltionssuspensionen enthalten beispielsweise wässrige oder alkoholische, bevorzugt ethanolische, gegebenenfalls ethanolische im Gemisch mit wässerigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent Ethanol. Die restlichen Volumenprozente werden von Wasser aufgefüllt. Die **1** und **2**, getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure. Bevorzugte anorganische Säuren sind Salzsäure und Schwefelsäure. Es können auch die Säuren verwendet werden, die gegebenenfalls bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.
Andere Ausführungsformen beinhalten diese Verbindung(en).
In einer solchen bevorzugten Ausführungsform liegt der Gehalt bezogen auf Natriumedetat unter 100 mg /100ml, bevorzugt unter 50 mg/100ml, besonders bevorzugt unter 20 mg/100ml. Generell sind solche Inhaltionslösungen bevorzugt, in denen der Gehalt an Natriumedetat bei 0 bis 10mg/100ml liegt.

Den erfindungsgemäßen treibgasfreien Inhaltionslösungen können Co-Solventien und/oder weitere Hilfsstoffe zugesetzt werden.
Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder physiologisch verträgliche Stoff verstanden, der kein Wirkstoff ist, aber zusammen mit dem (den) Wirkstoff(en) in dem physiologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, und/oder Vitamine Zu den Zusatzstoffen zählen auch physiologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhaltionslösungen sind besonders solche Inhalatoren, die eine kleine Menge einer flüssigen Formulierung in der therapeutisch notwendigen Dosierung binnen weniger Sekunden in ein therapeutischinhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 20 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, daß der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat® bekannt.

Dieser Vernebler (Respimat®) kann vorteilhaft zur Erzeugung der erfindungsgemäßen inhalierbaren Aerosole enthaltend die Wirkstoffkombination aus **1** und **2** eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4-insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf die dort offenbarte Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

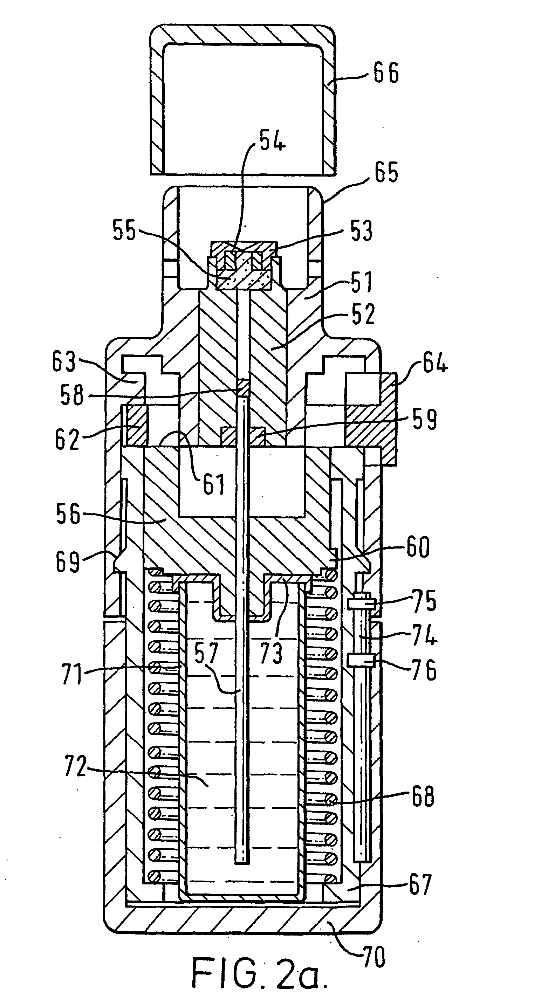

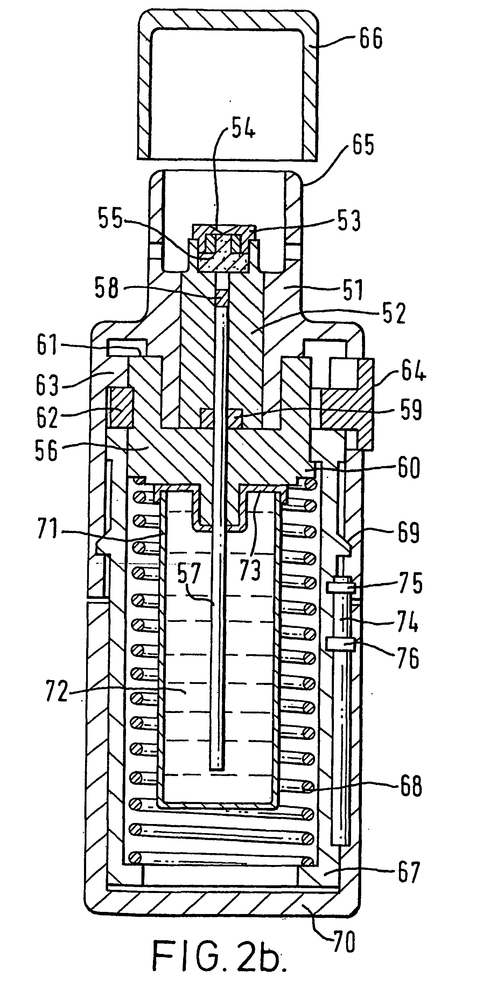

In den dieser Patentanmeldung beigefügten Figuren 2a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 2a zeigt einen Längsschnitt durch den Zerstäuber bei gespannter Feder, Figur 2b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inahalatoren, beispielsweise Jet-Stream-Inhalatoren, vernebelt werden.

Die erfindungsgemäßen treibgasfreien Inhalationslösungen oder Suspensionen können neben den vorstehend, zur Applikation im Respimat vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Die folgenden Beispiele dienen einer weitergehenden Erläuterung der vorliegenden Erfindung.

Nachfolgend wird zunächst die Darstellung von der im Rahmen der vorliegenden Erfindung zum Einsatz gelangenden Verbindungen **1** beschrieben, die noch nicht im Stand der Technik bekannt sind.

### Darstellung der Verbindungen der Formel 1:

### 1.a.: 2,2-Diphenylpropionsäurechlorid:.

Zu einer Suspension aus 25,0 g (0,11 mol) 2,2-Diphenylpropionsäure, 100 ml Dichlormethan und 4 Tropfen Dimethylformamid.werden bei 20° C 52,08g (0,33 mol) Oxalylchlorid langsam zugetropft. Es wird 1 h bei 20°C und 0,5 h bei 50° C gerührt. Das Lösungsmittel wird abdestilliert und der verbleibende Rückstand ohne weitergehende Reinigung in die nächste Stufe eingesetzt.

### 1.b.: 2,2-Diphenylpropionsäurescopinester:

Der aus Stufe 1.a. erhaltene Rückstand wird in 100 ml Dichlormethan gelöst und bei 40° C tropfenweise mit einer Lösung aus 51,45 g (0,33 mol) Scopin in 200 ml Dichlormethan versetzt. Die entstandene Suspension wird 24 h bei 40° C gerührt, anschließend der entstandene Niederschlag abgesaugt und das Filtrat zunächst mit Wasser, dann wässriger Salzsäure sauer extrahiert. Die vereinigten wässrigen Phasen werden mit wässriger Natriumcarbonatlösung alkalisch gestellt, mit Dichlormethan extrahiert, die organische Phase über Na₂SO₄ getrocknet, zur Trockene eingedampft und aus dem Rückstand das Hydrochlorid gefällt. Die Reinigung erfolgt durch Umkristallisation aus Acetonitril. Ausbeute: 20,85 g (= 47 % d. Th.)

DC: Rf-Wert: 0,24 (Laufmittel: sek. Butanol/Ameisensäure/Wasser 75:15:10); Smp.: 203-204°C.

### 1.c: 2,2-Diphenylpropionsäurescopinester-Methobromid :

11,98 g (0,033 mol) der Verbindung aus Stufe 1.b , 210 ml Acetonitril, 70 ml Dichlormethan und 20,16 g (0,1 mol) 46,92 % iges Brommethan in Acetonitril werden bei 20°C zusammengegeben und 3 Tage stehen gelassen. Die Lösung wird zur Trockene eingedampft und der Rückstand aus Isopropanol umkristallisiert.
Ausbeute: 11,34 g (= 75 % d. Th); Smp.: 208-209°C.
C₂₄H₂₈NO₃xBr (458,4);

| | | | | |
|---|---|---|---|---|
| Elementaranalyse | berechnet | C (62,89) | H (6,16) | N (3,06) |
| | gefunden. | C (62,85) | H (6,12) | N (3,07). |

In zu Stufe 1.3 analoger Art und Weise lassen sich jene Salze **1** erhalten, in denen X⁻ für ein anderes einfach negativ geladenes Anion als Bromid steht.

Die nachfolgenden Formulierungsbeispiele, die in Analogie zu im Stand der Technik an sich bekannten Verfahren erhalten werden können, dienen einer weitergehenden Erläuterung der vorliegenden Erfindung.

### Formulierungsbeispiele:

### A) Inhalationspulver

1)

| **Bestandteile** | **µg pro Kapsel** |
|---|---|
| **1'**-Bromid | 100 |
| Budesonid | 200 |
| Lactose | 4700 |
| **Summe** | 5000 |

2)

| **Bestandteile** | ***µ*****g pro Kapsel** |
|---|---|
| **1'**-Bromid | 100 |
| Fluticasone-propionat | 125 |
| Lactose | 4775 |
| **Summe** | 5000 |

3)

| **Bestandteile** | **µg pro Kapsel** |
|---|---|
| **1****'**-Bromid | 100 |
| Mometasone-furoat X H₂O | 250 |
| Lactose | 4650 |
| Summe | 5000 |

4)

| **Bestandteile** | **µg pro Kapsel** |
|---|---|
| **1****'**-Bromid | 100 |
| Ciclesonide | 250 |
| Lactose | 4650 |
| **Summe** | 5000 |

5)

| **Bestandteile** | **µg pro Kapsel** |
|---|---|
| **1**'-Bromid | 50 |
| Budesonid | 125 |
| Lactose | 4825 |
| **Summe** | 25000 |

6)

| **Bestandteile** | **µg pro Kapsel** |
|---|---|
| **1**'-Bromid | 50 |
| Fluticasone-propionat | 200 |
| Lactose | 4750 |
| **Summe** | 5000 |

7)

| **Bestandteile** | **µg pro Kapsel** |
|---|---|
| **1'**-Bromid | 75 |
| Mometasone-furoat X H₂O | 250 |
| Lactose | 4675 |
| **Summe** | 5000 |

8)

| **Bestandteile** | **µg pro Kapsel** |
|---|---|
| **1****'**-Bromid | 75 |
| Ciclesonide | 250 |
| Lactose | 4675 |
| **Summe** | 5000 |

9)

| **Bestandteile** | **µg pro Kapsel** |
|---|---|
| **1'**-Bromid | 100 |
| ST-126 | 250 |
| Lactose | 4650 |
| **Summe** | 5000 |

10)

| **Bestandteile** | **µg pro Kapsel** |
|---|---|
| **1'**-Bromid | 50 |
| ST-126 | 125 |
| Lactose | 4825 |
| **Summe** | 5000 |

### B) Treibgashaltige Inhaltionsaerosole:

1) Suspensionsaerosol:

| **Bestandteile** | **Gew-%** |
|---|---|
| **1**'-Bromid | 0,020 |
| Budesonid | 0,4 |
| Sojalecithin | 0,2 |
| TG 134a : TG227 = 2:3 | ad 100 |

2) Suspensionsaerosol:

| **Bestandteile** | **Gew- %** |
|---|---|
| **1**'-Bromid | 0,020 |
| Fluticasone-propionat | 0,3 |
| Isopropylmyristat | 0,1 |
| TG 227 | ad 100 |

3) Suspensionsaerosol:

| **Bestandteile** | **Gew-%** |
|---|---|
| **1'****-**Bromid | 0,020 |
| Mometasone-furoat X H₂O | 0,6 |
| Isopropylmyristat | 0,1 |
| TG 227 | ad 100 |

4) Suspensionsaerosol:

| **Bestandteile** | **Gew-%** |
|---|---|
| **1**'-Bromid | 0,020 |
| Ciclesonide | 0,4 |
| Isopropylmyristat | 0,1 |
| TG 134a : TG227 = 2:3 | ad 100 |

5) Lösungsaerosol:

| **Bestandteile** | **Gew- %** |
|---|---|
| **1****'**-Bromid | 0,039 |
| Budesonid | 0,4 |
| Ethanol, absolut | 0,5 |
| Isopropylmyristat | 0,1 |
| TG 134a : TG227 = 2:3 | ad 100 |

6) Lösungsaerosol:

| **Bestandteile** | **Gew- %** |
|---|---|
| **1'**-Bromid | 0,039 |
| Fluticasone-propionat | 0,3 |
| Ethanol, absolut | 0,5 |
| Isopropylmyristat | 0,1 |
| TG 134a : TG227 = 2:3 | ad 100 |

7) Lösungsaerosol:

| **Bestandteile** | **Gew-%** |
|---|---|
| **1**'-Bromid | 0,039 |
| Mometasone-furoat X H₂O | 0,6 |
| Ethanol, absolut | 0,5 |
| Isopropylmyristat | 0,1 |
| TG 134a : TG227 = 2:3 | ad 100 |

8) Lösungsaerosol:

| **Bestandteile** | **Gew-%** |
|---|---|
| **1**'-Bromid | 0,039 |
| Ciclesonide | 0,4 |
| Ethanol, absolut | 0,5 |
| Isopropylmyristat | 0,1 |
| TG 134a : TG227 = 2:3 | ad 100 |

9) Lösungsaerosol:

| **Bestandteile** | **Gew-%** |
|---|---|
| **1****'**-Bromid | 0,039 |
| ST-126 | 0,6 |
| Ethanol, absolut | 0,5 |
| Isopropylmyristat | 0,1 |
| TG 134a : TG227 = 2:3 | ad 100 |

10) Lösungsaerosol:

| **Bestandteile** | **Gew-%** |
|---|---|
| **1**'-Bromid | 0,039 |
| ST-126 | 0,4 |
| Ethanol, absolut | 0,5 |
| Isopropylmyristat | 0,1 |
| TG 134a : TG227 = 2:3 | ad 100 |

## Patentansprüche

1. Arzneimittel **gekennzeichnet durch** einen Gehalt an einem oder mehreren, bevorzugt einem Salz der Formel **1** 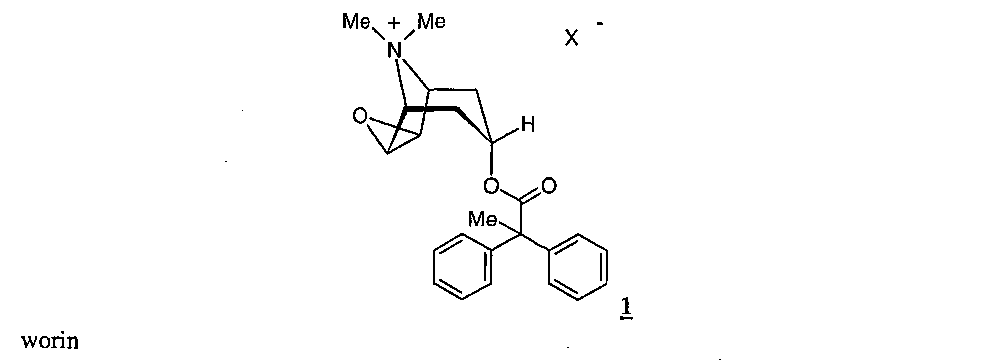
X⁻ ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat bedeutet,
in Kombination mit einem oder mehreren, bevorzugt einem Steroid **2**, gegebenenfalls in Form ihrer Diastereomere, Gemische der Diastereomere oder in Form der Racemate, gegebenenfalls in Form der Solvate oder Hydrate sowei gegebenenfalls gemeinsam mit einem pharmazeutisch verträglichen Hilfsstoff.

2. Arzneimittel nach Anspruch 1, **dadurch gekennzeichnet, daß** die Wirkstoffe **1** und **2** entweder gemeinsam in einer einzigen Darreichungsform oder in zwei getrennten Darreichungsformen enthalten sind.

3. Arzneimittel nach Anspruch 1 oder 2, **dadurch gekennzeichnet, daß** es sich bei den Steroiden **2** um Flunisolide, Beclomethasone, Triamcinolone, Budesonid, Fluticasone, Mometasone, Ciclesonide, Rofleponide, ST-126 oder Dexametasone handelt.

4. Arzneimittel nach Anspruch 3, **dadurch gekennzeichnet, daß** es sich bei den Steroiden **2** um Budesonid, Fluticasone, Mometasone, Ciclesonide oder ST-126 handelt.

5. Arzneimittel nach einem der Ansprüche 1 bis 4, **dadurch gekennzeichnet, daß** die Gewichtsverhältnisse von **1****'** 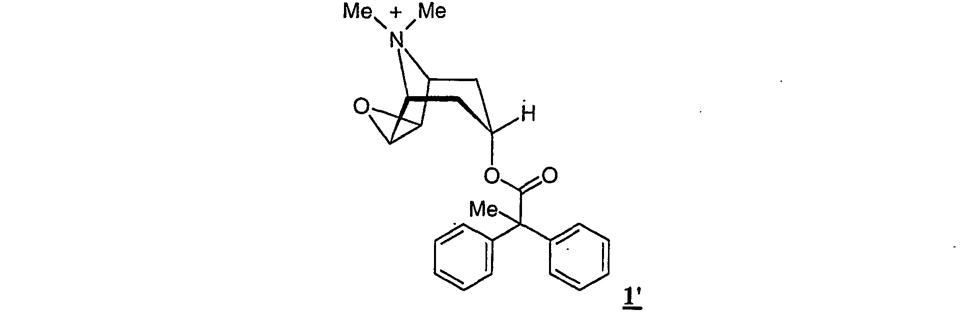 zu Steroid **2** in einem Bereich von 1:250 bis 250:1, bevorzugt in einem Bereich von 1:150 bis 150:1 liegen.

6. Arzneimittel nach einem der Ansprüche 1 bis 5, **dadurch gekennzeichnet, daß** es in Form einer für die Inhalation geeigneten Darreichungsform vorliegt.

7. Arzneimittel nach Anspruch 6, **dadurch gekennzeichnet, daß** es sich um eine Darreichungsform ausgewählt aus der Gruppe Inhalationspulver, treibgashaltige Dosieraerosole und treibgasfreie Inhalationslösungen oder -suspensionen handelt.

8. Arzneimittel nach Anspruch 7, **dadurch gekennzeichnet, daß** es ein Inhalationspulver ist, welches **1** und **2** im Gemisch mit geeigneten physiologisch unbedenkliche Hilfsstoffen ausgewählt aus der Gruppe bestehend aus Monosacchariden, Disacchariden, Oligo- und Polysacchariden, Polyalkoholen, Salzen, oder Mischungen dieser Hilfsstoffe enthält.

9. Inhalationspulver nach Anspruch 8, **dadurch gekennzeichnet, daß** der Hilfsstoff eine maximale mittlere Teilchengröße von bis zu 250µm, bevorzugt zwischen 10 und 150µm aufweist.

10. Arzneimittel nach Anspruch 7, **dadurch gekennzeichnet, daß** es ein Inhalationspulver ist, welches als Bestandteile lediglich die Wirkstoffe **1** und **2** enthält.

11. Arzneimittel nach Anspruch 7, **dadurch gekennzeichnet, daß** es sich um ein treibgashaltiges Inhalationsaerosol handelt, welches **1** und **2** in gelöster oder dispergierter Form enthält.

12. Treibgashaltiges Inhalationsaerosol nach Anspruch 11, **dadurch gekennzeichnet, daß** es als Treibgas Kohlenwasserstoffe wie n-Propan, n-Butan oder Isobutan oder Halogenkohlenwasserstoffe wie chlorierte und/oder fluorierte Derivate des Methans, Ethans, Propans, Butans, Cyclopropans oder Cyclobutans enthält.

13. Treibgashaltiges Inhaltionsaerosol nach Anspruch 12, **dadurch gekennzeichnet, daß** das Treibgas TG11, TG12, TG134a, TG227 oder Gemische davon, bevorzugt TG134a, TG227 oder ein Gemisch davon darstellt.

14. Treibgashaltiges Inhalationsaerosol nach Anspruch 11, 12 oder 13, **gekennzeichnet**, daß es bis zu 5 Gew-% an Wirkstoff **1** und **2** enthalten kann.

15. Arzneimittel nach Anspruch 7, **dadurch gekennzeichnet, daß** es sich um eine treibgasefreie Inhalationslösung handelt, die als Lösemittel Wasser, Ethanol oder ein Gemisch aus Wasser und Ethanol enthält.

16. Inhalationslösung nach Anspruch 15, **dadurch gekennzeichnet, daß** sie gegebenenfalls weiter Co-Solventien und/oder Hilfsstoffe enthält.

17. Inhalationslösung nach Anspruch 16, **dadurch gekennzeichnet, daß** sie als Co-Solventien Bestandteile enthalten, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

18. Inhalationslösungen nach einem der Ansprüche 16 oder 17, **dadurch gekennzeichnet, daß** sie als Hilfsstoffe oberflächenaktive Stoffe, Stabilisatoren, Komplexbildner, Antioxidantien und/oder Konservierungsstoffe, Geschmackstoffe und/oder Vitamine enthalten.

19. Inhalationslösungen nach Anspruch 18, **dadurch gekennzeichnet, daß** sie als Komplexbildner Editinsäure oder ein Salz der Editinsäure, bevorzugt Natriumedetat, enthalten.

20. Verwendung einer Zusammensetzung nach einem der Ansprüche 1 bis 19 zur Herstellung eines Medikaments zur Behandlung von entzündlichen und/oder obstruktiven Atemwegserkrankungen, insbesondere von Asthma oder COPD.

## Claims

1. Pharmaceutical compositions, **characterised in that** they contain one or more, preferably one, salt of formula **1** 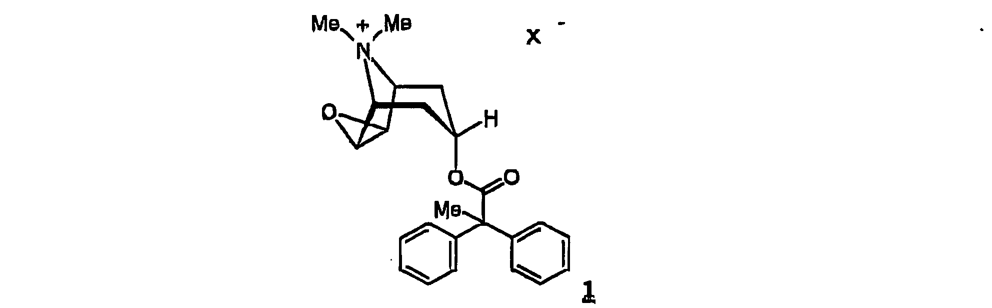 wbgrein
X⁻ denotes an anion with a single negative charge, preferably an anion selected from the group consisting of chloride, bromide, iodide, sulphate, phosphate, methanesulphonate, nitrate, maleate, acetate, citrate, fumarate, tartrate, oxalate, succinate, benzoate and p-toluenesulphonate,
combined with one or more, preferably one, steroid **2**, optionally in the form of the diastereomers, mixtures of the diastereomers or in the form of the racemates thereof, optionally in the form of the solvates or hydrates and optionally together with a pharmaceutically acceptable excipient.

2. Pharmaceutical composition according to claim 1, **characterised in that** the active substances **1** and **2** are present either together in a single formulation or in two separate formulations.

3. Pharmaceutical composition according to claim 1 or 2, **characterised in that** the steroids **2** are flunisolide, beclomethasone, triamcinolone, budesonide, fluticasone, mometasone, ciclesonide, rofleponide, ST-126 or dexamethasone.

4. Pharmaceutical composition according to claim 3, **characterised in that** the steroids **2** are budesonide, fluticasone, mometasone, ciclesonide or ST-126.

5. Pharmaceutical composition according to one of claims 1 to 4,
**characterised in that** the weight ratios of **1****'** 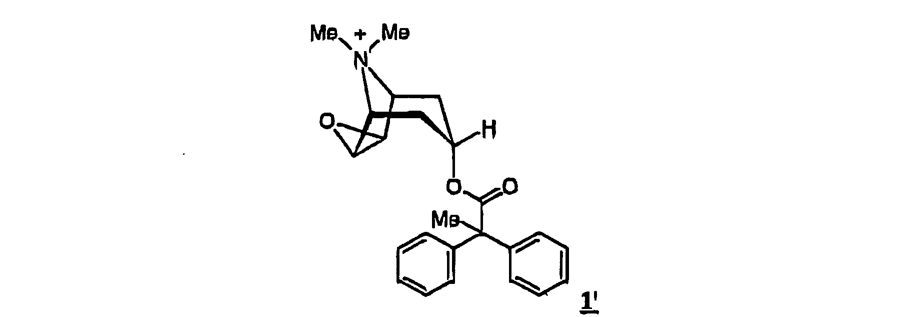 to steroid **2** are in the range from 1:250 to 250:1, preferably in the range from 1:150 to 150:1.

6. Pharmaceutical composition according to one of claims 1 to 5,
**characterised in that** it is in the form of a preparation suitable for inhalation.

7. Pharmaceutical composition according to claim 6, **characterised in that** it is a preparation selected from among the inhalable powders, propellant-containing metered-dose aerosols and propellant-free inhalable solutions or suspensions.

8. Pharmaceutical composition according to claim 7, **characterised in that** it is an inhalable powder which contains **1** and **2** in admixture with suitable physiologically acceptable excipients selected from among the monosaccharides, disaccharides, oligo- and polysaccharides, polyalcohols, salts, or mixtures of these excipients.

9. Inhalable powder according to claim 8, **characterised in that** the excipient has a maximum average particle size of up to 250µm, preferably between 10 and 150µm.

10. Pharmaceutical composition according to claim 7, **characterised in that** it is an inhalable powder which contains only the active substances **1** and **2** as its ingredients.

11. Pharmaceutical composition according to claim 7, **characterised in that** it is a propellant-containing inhalable aerosol which contains **1** and **2** in dissolved or dispersed form.

12. Propellant-containing inhalable aerosol according to claim 11,
**characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

13. Propellant-containing inhalable aerosol according to claim 12,
**characterised in that** the propellant gas is TG11, TG12, TG134a, TG227 or mixtures thereof, preferably TG134a, TG227 or a mixture thereof.

14. Propellant-containing inhalable aerosol according to claim 11, 12 or 13,
**characterised in that** it may contain up to 5 % by weight of active substance **1** and **2**,

15. Pharmaceutical composition according to claim 7, **characterised in that** it is a propellant-free inhalable solution which contains water, ethanol or a mixture of water and ethanol as solvent.

16. Inhalable solution according to claim 15, **characterised in that** it optionally contains other co-solvents and/or excipients.

17. Inhalable solution according to claim 16, **characterised in that** it contains as co-solvents ingredients which contain hydroxyl groups or other polar groups, e.g. alcohols - particularly isopropyl alcohol, glycols - particularly propyleneglycol, polyethyleneglycol, polypropyloneglycol, glycolether, glycerol, polyoxyethylene alcohols and polyoxyethylene fatty acid esters.

18. Inhalable solutions according to one of claims 16 or 17, **characterised in that** they contain as excipients surfactants, stabilisers, complexing agents, antioxidants and/or preservatives, flavourings and/or vitamins.

19. Inhalable solutions according to claim 18, **characterised in that** they contain as complexing agents editic acid or a salt of editic acid, preferably sodium edetate.

20. Use of a composition according to one of claims 1 to 19 for preparing a medicament for the treatment of inflammatory and/or obstructive respiratory complaints, particularly asthma or COPD.

## Revendications

1. Médicament **caractérisé par** une teneur en un ou plusieurs, de préférence un sel de formule **1** 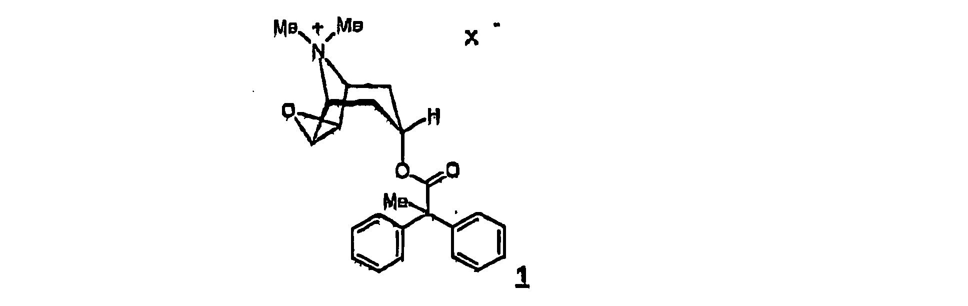 où
X⁻ représente un anion à une seule charge négative, de préférence un anion choisi dans le groupe consistant en chlorure, bromure, iodure, sulfate, phosphate, méthanesulfonate, nitrate, maléate, acétate, citrate, fumarate, tartrate, oxalate, succinate, benzoate et p-toluénesulfonate,
en combinaison avec un ou plusieurs, de préférence un stéroïde **2**, éventuellement sous forme de leurs diastéréoisomères, de mélanges des diastéréoisomères ou sous forme des racémates, éventuellement sous forme des solvates ou des hydrates ainsi éventuellement que conjointement avec un adjuvant pharmaceutiquement acceptable.

2. Médicament selon la revendication 1 **caractérisé en ce que** les principes actifs **1** et **2** sont contenus soit ensemble dans une seule forme d'administration soit dans deux formes d'administration séparées.

3. Médicament selon la revendication 1 ou 2 **caractérisé en ce que** les steroïdes **2** sont le flunipolicle, la beclométhasone, la triamcinolone, le budésonide, la fluticasone, la mométasone, le ciclésonide, le rofléponide, ST-126 ou la dexamétasone.

4. Médicament selon la revendication 3 **caractérisé en ce que** les stëroïdes **2** sont le budésonide, la fluticasone, la mométasone, le ciclésopide ou ST-126.

5. Médicament selon l'une des revendications 1 à 4 **caractérisé en ce que** les proportions massiques de **1****'** 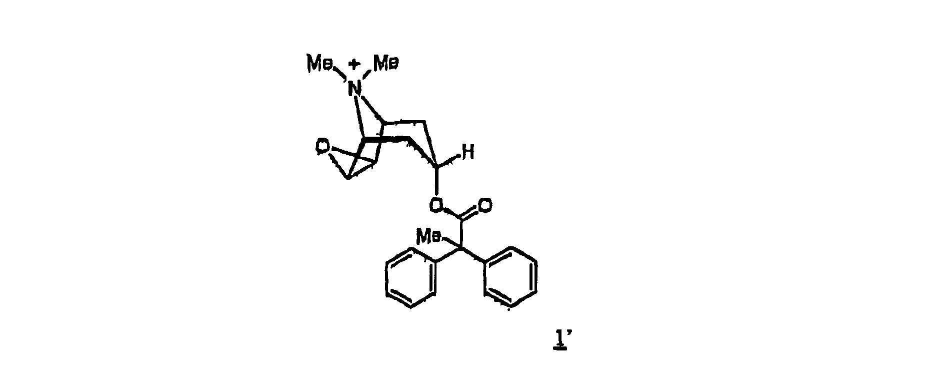 par rapport au stéroïde **2** sont situées dans une plage de 1 : 250 à 250 : 1, de préférence dans une plage de 1 : 150 à 150 : 1.

6. Médicament selon l'une des revendications 1 à 5 **caractérisé en ce qu'**il est sous forme d'une forme d'administration appropriée à l'inhalation.

7. Médicament selon la revendication 6 **caractérisé en ce qu'**il s'agit d'une forme d'administration choisie dans le groupe poudre pour inhalation, aérosols de dosage contenant un gaz propulseur et solutions ou suspensions pour inhalation sans gaz propulseur.

8. Médicament selon la revendication 7 **caractérisé en ce que** c'est une poudre pour inhalation qui contient **1** et **2** en mélange avec des adjuvants physiologiquement acceptables appropriés choisis dans le groupe consistant en les monosaccharides, les disaccharides, les oligo- et polysaccharides, les polyalcools, les sels ou des mélanges de ces adjuvants.

9. Poudre pour inhalation selon la revendication 8 **caractérisée en ce que** l'adjuvant présente une dimension de particule moyenne maximale de jusqu'à 250 µm, de préférence entre 10 et 150 µm.

10. Médicament selon la revendication 7 **caractérisé en ce que** c'est une poudre pour inhalation qui contient comme constituants uniquement les principes actifs **1** et **2****.**

11. Médicament selon la revendication 7 **caractérisé en ce qu'**il s'agit d'un aérosol pour inhalation contenant un gaz propulseur qui contient **1** et **2** sous forme dissoute ou dispersée.

12. Aérosol pour inhalation contenant un gaz propulseur selon la revendication 11 **caractérisé en ce qu'**il contient comme gaz propulseur des hydrocarbures comme le n-propane, le n-butane ou l'isobutane ou des halogénohydrocarbures comme des dérivés chlorés et/ou fluorés du méthane, de l'éthane, du propane, du butane, du cyclopropane ou du cyclobutane.

13. Aérosol pour inhalation contenant un gaz propulseur selon la revendication 12 catactérisé en ce que le gaz propulseur est TG11, TG12, TG134a, TG227 ou des mélanges de ceux-ci, de préférence TG134a, TG227 ou un mélange de ceux-ci.

14. Aérosol pour inhalation contenant un gaz propulseur selon la revendication 11, 12 ou 13 **caractérise en ce qu'**il peut contenir jusqu'à 5 % en masse de principe actif **1** et **2**.

15. Médicament selon la revendication 7 **caractérisé en ce qu'**il s'agit d'une solution pour inhalation sans gaz propulseur qui contient comme solvant de l'eau, de l'éthanol ou un mélange d'eau et d'éthanol.

16. Solution pour inhalation selon la revendication 15 **caractérisée en ce qu'**elle contient éventuellentent en outre des co-solvants et/ou adjuvants.

17. Solution pour inhalation selon la revendication 16 **caractérisée en ce qu'**elle contient comme co-solvants des constituants qui contiennent des groupes hydroxyle ou d'autres groupes polaires, par exemple des alcools - en particulier l'alcool isopropylique, des glycols - en particulier le propylèneglycol, le polyéthylèneglycol, le polypropylèneglycol, un éther de glycol, le glycérol, des polyoxyéthylène-alcools et des polyoxyéthylène-esters d'acides gras.

18. Solutions pour inhalation selon l'une des revendications 16 ou 17 **caractérisées en ce qu'**elles contiennent comme adjuvants des substances tensioactives, des stabilisants, des complexants, des antioxydants et/ou des conservateurs, des agents de sapidité et/ou des vitamines.

19. Solutions pour inhalation selon la revendication 18 **caractérisées en ce qu'**elles contiennent comme complexant l'acide édétique on un sel d'acide édétique, de préférence l'édétate de sodium.

20. Utilisation d'une composition selon l'une des revendications 1 à 19 pour la production d'un médicament pour le traitement des maladies inflammatoires et/ou obstructives des voies respiratoires, en particulier de l'asthme ou de la COPD.
